# EUROPÄISCHE PATENTSCHRIFT

(11) **EP 1 420 726 B1**
(45) Veröffentlichungstag und Bekanntmachung des Hinweises auf die Patenterteilung: **09.03.2011**
(21) Anmeldenummer: 02767119.7
(22) Anmeldetag: 23.08.2002
(51) Int. Cl.: A61F 2/60, A61F 2/80

(54) **PROTHESENSCHAFT MIT DICHTUNG**
PROSTHESIS SHAFT COMPRISING A SEAL
TIGE DE PROTHESE DOTEE D'UN JOINT

(30) Priorität: 30.08.2001 DE 10142492
(43) Veröffentlichungstag der Anmeldung: 26.05.2004
(73) Patentinhaber: Ossur HF, 110 Reykjavik (IS)
(72) Erfinder: CARSTENS, Felix, 67433 Neustadt/Weinstrasse (DE)
(74) Vertreter: Rüger, Barthelt & Abel Patentanwälte
(86) Internationale Anmeldenummer: PCT/DE2002/003085
(87) Internationale Veröffentlichungsnummer: WO 2003/024370

(56) Entgegenhaltungen:
- EP-A- 0 631 765
- DE-A- 2 060 239
- DE-U- 9 419 208
- US-A- 1 398 824
- US-A- 1 893 853
- US-A- 2 634 424
- US-A- 5 314 496

## Beschreibung

Der Amputationsstumpf sitzt nach Art eines Kolbens in dem becherförmigen Prothesenschaft. Also ist es eine bekannte Maßnahme den äußeren Luftdruck dazu zu verwenden, den Prothesenschaft am Stumpf festzuhalten. Hierzu muss der Stumpf gegen die Innenwand des Prothesenschaftes oder einen auf den Stumpf aufgezogenen Liner abgedichtet gehalten sein.

Eine Kraft die bestrebt wäre den Prothesenschaft vom Stumpf abzuziehen, ruft bei hinreichender Abdichtung umgehend einen Unterdruck hervor, der der abziehenden Kraft entgegenwirkt. Allerdings bricht die Haltekraft sofort zusammen, sobald in den Spaltraum zwischen dem Amputationsstumpf und dem Prothesenschaft Luft eindringt.

Ein Prothesenschaft gemäß den Merkmalen des oberbegriffs des Anspruchs 1 und ein Liner gemäß den Merkmalen des oberbegriffs des Anspruchs 13 ist jeweils aus der Druckschrift US-A- 2634 424 bekannt.

Aus der EP 0 631 765 ist ein Prothesenschaft bekannt, der mit einem Dichtmittel versehen ist, das dazu dient, die Abdichtung zwischen dem Stumpf und dem Prothesenschaft zu gewährleisten.

Der Prothesenschaft weist hierzu im Abstand zu seinem distalen Ende eine umlaufende Nut auf, in die ein Dichtungsring eingelegt ist. Der Dichtungsring besteht aus einem ringförmigen Kern, der mit einer elastomeren Masse umspritzt ist. Zum Innenraum des Prothesenschaftes hin ist an dem Ring eine Dichtlippe ausgebildet.

Mit Hilfe der Dichtlippe, die sich an die Haut des Stumpfes anschmiegt, sollen Durchmesserschwankungen des Stumpfes ausgeglichen werden. Die lichte Weite des Prothesenschaftes ist, da er aus einem starren Material besteht, unveränderlich. Das Volumen des Stumpfes ändert sich hingegen langfristig und auch kurzfristig. Die langfristigen Schwankungen ergeben sich aufgrund von Änderungen des Gewebevolumens, während die kurzfristigen Schwankungen beispielsweise vom Blutdruck und der Durchblutung des Stumpfes abhängig sind. Es ist bekannt, dass bei hohen Temperaturen die Extremitäten zum Anschwellen neigen, während ihr Durchmesser bei tiefen Temperaturen abnimmt. Auch der gesunde Mensch kann dies leicht an seinem Uhrenarmband beobachten.

Wenn der Patient den Amputationsstumpf in den Prothesenschaft einsteckt, wird die Lippendichtung in Richtung auf das geschlossene distale Ende des Prothesenschaftes umgeklappt. Die Lippendichtung liegt allein aufgrund ihrer Vorspannung an dem Stumpf an.

Sobald eine Kraft auftritt, die den Prothesenschaft vom Stumpf abzuziehen bestrebt ist, wirkt zwischen dem Stumpf und dem Raum innerhalb des Prothesenschaftes zwischen dem distalen Ende und der Lippendichtung ein geringfügiger Unterdruck. Die Größe des Unterdrucks ist der Vergrößerung des Spaltvolumens zwischen Stumpf und Prothesenschaft etwa proportional.

Bei der bekannten Anordnung hat der Druckgradient an der Lippendichtung eine Richtung, die bestrebt ist, die Lippendichtung von dem Stumpf abzuheben, womit Luft in den Spaltraum eindringen kann und die Haltewirkung durch Unterdruck zusammenbrechen lässt.

Wie groß der zulässige Unterdruck ist, bei dem das unerwünschte Abheben der Dichtlippe auftritt, hängt von der radialen Vorspannung ab, mit der die Dichtlippe am Stumpf anliegt. Es hat sich gezeigt, dass eine verhältnismäßig große radiale Vorspannung erforderlich ist, die wiederum zu Durchblutungsstörungen im Bereich der Dichtlippe sowie in dem Bereich zwischen der Dichtlippe und dem distalen Ende des Stumpfes führen kann.

Die DE 20 60 239 A zeigt einen einen Stumpf aufnehmenden becherförmiger Prothesenschaft mit einer im wesentlichen formstabilen Schaftwand. Die Schaftwand bildet eine nach außen weisende Außenseite sowie eine nach innen weisende Innenseite bildet. Ein im wesentlichen formstabilen Schaftboden ist mit der Schaftwand verbunden ist und begrenzt zusammen mit der Schaftwand einen becherförmigen Innenraum.

Der Innenraum ist zum Einführen des Stumpfes über eine Einführöffnung von außen zugänglich, die von einem Schaftrand begrenzt ist. Die Gestalt des Innenraums ist an die Art des Stumpfes und die Art der prothetischen Versorgung angepasst. Am unteren Ende des Innenraums ist eine zum Innenraum abgedichtete Kammer vorgesehen, die sich längs dem Umfang des Innenraums als Ring erstreckt.

Die Kammer weist eine zum Innenraum des Prothesenschaftes gelegene Wand aus einem anschmiegsamen luftundurchlässigen Material auf. Aus der Kammer führt eine Kanal nach außen, der mit einer Pumpe versehen ist, um die Kammer bei Gebrauch des Prothesenschafts auf zu pumpen.

Ausgehend hiervon ist es Aufgabe der Erfindung, einen Prothesenschaft bzw. einen Liner zu schaffen, die eine gute Abdichtwirkung gegenüber dem Stumpf bzw. einem Prothesenschaft gewährleisten, ohne dass nennenswerte Vorspannkräfte erforderlich sind.

Diese Aufgabe wird erfindungsgemäß mit dem Prothesenschaft nach dem Anspruch 1 beziehungsweise dem Liner nach dem Anspruch 13 gelöst.

Der erfindungsgemäße Prothesenschaft ist in der Nähe seiner proximalen Öffnung, also im Abstand vom distalen geschlossenen Ende mit einer ringförmigen Kammer versehen, die sich vorzugsweise längs dem gesamten Innenumfang des becherförmigen Innenraums des Prothesenschaftes erstreckt. Sie verläuft sozusagen wie ein Ring an der Innenseite des Prothesenschaftes und folgt dabei dessen Kontur.

Wie bei Prothesenschäften üblich, ist die Kontur des Innenraums sowie sein Volumen an die Art des Schaftes und die Art der prothetischen Versorgung angepasst.

Die Kammer weist eine zu dem Innenraum des Prothesenschaftes hin gelegene Wand aus einem anschmiegsamen Material auf. Diese Wand soll sich abdichtend an die Haut des Stumpfes oder im Falle der Verwendung eines Liners an die Außenseite des Liners anlegen. Ein Druckausgleichskanal sorgt dafür, dass in der Kammer ständig wenigstens Atmosphärendruck herrscht.

Wenn bei der erfindungsgemäßen Anordnung eine Kraft auftritt, die den Prothesenschaft vom Stumpf abzieht, entsteht wiederum in dem Spalt zwischen dem Stumpf und dem Prothesenschaft im Bereich zwischen der Kammer und dem distalen Ende ein Unterdruck. Wegen der Belüftung der Kammer mit Atmosphärendruck wirkt der Druckgradient an der Wand in einer Weise, dass er bestrebt ist, die anschmiegsame Wand dichter und fester an die Haut des Stumpfes beziehungsweise den Liner anzupressen.

Im Gegensatz zum Stand der Technik bewirkt also bei der erfindungsgemäßen Lösung der bestehende Unterdruck eine Erhöhung der Anpresskraft und somit eine Verbesserung der Dichtwirkung.

Bei der erfindungsgemäßen Lösung ist die Dichtwirkung bei auftretendem Unterdruck nicht mehr von der Vorspannung des abdichtenden Elementes abhängig. Vielmehr stellt sich bei der erfindungsgemäßen Lösung die abdichtende Kraft unterdruckabhängig selbstätig ein und verstärkt sich mit zunehmendem Unterdruck.

Es ist möglich die anschmiegsame Wand mit nur sehr geringer Ruhekraft an dem Stumpf anliegend zu halten.

Um die erforderliche Grundvorspannung zu erzeugen, kommen grundsätzlich mehrere Maßnahmen in Frage.

Eine Maßnahme besteht darin, die anschmiegsame Wand aus einem elastischen Material herzustellen und zwar als manschettenförmiges Gebilde, mit einem Durchmesser kleiner als es der lichten Weite des Prothesenschaftes an der Stelle der gewünschten Kammer entspricht. Wenn nun die Ränder dieser Manschette mit der Innenseite des Prothesenschaftes verbunden werden, muss das Material gereckt werden, während der Zwischenbereich im wesentlichen ungedehnt bleibt. Hierdurch wird die notwendige Vorspannung nach innen zu erzielt.

Eine andere Möglichkeit besteht darin in der Kammer einen Ring einzulegen, der die anschmiegsame Wand entsprechend nach innen zu formt.

Schließlich ist es denkbar, den Hauptbereich des Innenraums der Kammer in der Wand des Prothesenschaftes selbst unterzubringen und zwar in Gestalt einer an der Innenseite des Prothesenschaftes umlaufenden Nut. Die Nut ist zum Innenraum des Prothesenschaftes hin durch eine die glatte Kontur des Innenwandschaftes fortsetzende flexible und luftdichte Wand überbrückt ist.

Der Druckausgleichskanal ist jeweils im einfachsten Falle eine einfache Bohrung, die durch die Schaftwand führt, um den Innenraum der Kammer mit der Außenatmosphäre zu verbinden.

Der gleiche Grundgedanke lässt sich auch an einem Liner verwirklichen. In diesem Falle wird der Liner mit einer an der Außenseite umlaufenden Kammer versehen, die sich im Abstand zum distalen Ende des Liners befindet. Die Kammer ist wiederum mit einer anschmiegsamen Wand versehen, die jetzt jedoch auf der Außenseite des Liners vorgesehen ist und nach außen weist. Sie soll bei angezogenem Prothesenschaft gegen die Innenwand des Prothesenschaftes abdichten. Die Kammer ist wiederum über einen Druckausgleichskanal mit der Atmosphärendruck belüftet. Der Druckausgleichskanal verläuft in der Wand des Liners zu dessen proximalen Rand und ist so gestaltet, dass er auch bei angezogenem Liner und angezogenem Prothesenschaft nicht zugedrückt wird

Es versteht sich, dass die oben erläuterten neuen Dichtmaßnahmen auch bei solchen Schäften eingesetzt werden können, die zusätzlich mechanische Fixierungen aufweisen. So ist es nicht ausgeschlossen, den mit der Kammer versehenen Prothesenschaft in Verbindung mit einen Liner zu verwenden, der am distalen Ende über mechanische Rasteinrichtungen verfügt und formschlüssig mit dem Prothesenschaft verbunden ist, vorausgesetzt, die mechanische Rasteinrichtung ist hinreichend dicht. Außerdem kann die erfindungsgemäße kammerförmige Abdichtung bei Unterschenkelprothesen oder Armprothesen verwendet werden, bei denen Teile des Prothesenschaftes über die betreffenden Kondylen ragen, die zangenartig übergriffen werden.

Schließlich muss die Kammer nicht notwendigerweise konstant auf der selben Höhe innerhalb des Prothesenschaftes oder an der Außenseite des Liners verlaufen. Sie kann durchaus einen wellenförmigen Verlauf haben und somit an jenen Stellen angeordnet sein, die eine optimale Abdichtung gewährleisten.

Wenn durch besondere anatomische Verhältnisse gewährleistet ist, dass bestimmte Bereich des Stumpfes immer abgedichtet an der Schaftwand anliegen, braucht sich die abdichtende Kammer auch nicht über den gesamten Innenumfang des Schaftes oder Außenumfang des Liners zu erstrecken.

Im übrigen sind Weiterbildungen der Erfindung Gegenstand von Unteransprüchen. Dabei sollen auch solche Kombinationen als beansprucht angesehen werden, auf die kein ausdrückliches Ausführungsbeispiel gerichtet ist.

In der Zeichnung sind Ausführungsbeispiele des Gegenstandes der Erfindung dargestellt. Es zeigen:
- Fig. 1: eine Oberschenkelbeinprothese mit dem erfindungs- gemäßen Prothesenschaft im Längsschnitt,
- Fig. 2: das Zusammenwirken des erfindungsgemäßen Prothesen- schaftes mit einem Oberschenkelstumpf,
- Fig. 3: einen erfindungsgemäßen Prothesenschaft mit einer Ringnut, im Längsschnitt, und
- Fig. 4: einen erfindungsgemäßen Liner in Verbindung mit ei- nem Oberschenkelstumpf im Längsschnitt.

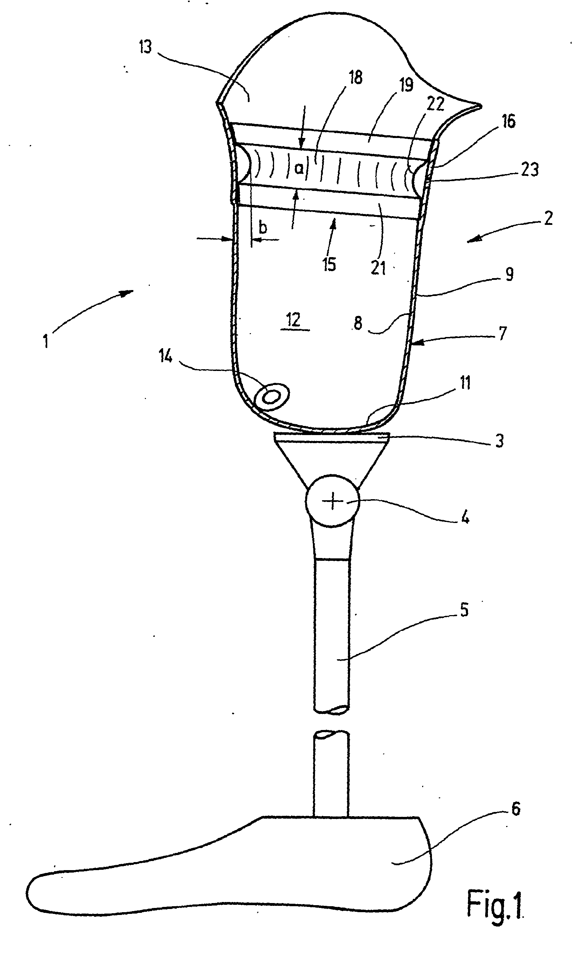

Fig. 1 zeigt in stark schematisierter Form als Beispiel für den Einsatz der Erfindung eine Oberschenkelbeinprothese 1. Zu der Prothese 1 gehört ein Prothesenschaft 2, der über einen Adapter 3 mit einer Kniegelenknachbildung 4 verbunden ist. An die Kniegelenknachbildung 4 schließt sich eine Unterschenkelnachbildung mit einem daran befestigten Fuß 6 an.

Der Prothesenschaft 2 setzt sich aus einer Schaftwand 7 mit einer Innenseite 8 und einer Außenseite 9 sowie einem Schaftboden 11 zusammen. Der Schaftboden 11 geht am distalen Ende der Schaftwand 7 einstückig in diese über. Der Schaftboden 11 begrenzt zusammen mit der Schaftwand 8 einen im Wesentlichen becherförmigen Innenraum 12, der über eine Einstecköffnung am proximalen Ende zugänglich ist. Die Länge des Innenraums 12 und dessen Kontur richtet sich nach der Art des Stumpfes und der Art der prothetischen Versorgung. Die spezielle Art des Innenraumes 12 ist hier nicht weiter von Bedeutung.

Der Prothesenschaft 2 ist ein sogenannter Saugschaft, der mit Hilfe von Unterdruck am amputierten Stumpf festgehalten wird.

Damit beim Einsteigen in den Prothesenschaft 2 vor dem Stumpf die Luft entweichen kann, ist im Übergangsbereich zwischen der Schaftwand 7 und dem Boden 11 ein Wahlweise zu betätigendes Ventil 14 vorgesehen.

Saugschäfte halten im Wesentlichen durch Unterdruck am Stumpf. Um die Aufrechterhaltung des Unterdrucks zu gewährleisten, ist nahe der proximalen Einstecköffnung 13 und somit im Abstand von dem Schaftboden 11 eine Kammer 15 vorgesehen. Die Kammer 15 erstreckt sich als geschlossener Ring längs dem Umfang der Schaftwand 7 auf deren Innenseite 8. Die Kammer 15 umgibt somit ringförmig den Innenraum 12.

Eine der Kammerwände ist ein entsprechender ringförmiger Bereich 16 der Schaftwand 7, während die nach innen zu gelegene Wand von einem ringförmigen Band 18 aus einem elastischen Material begrenzt. Das Band 18 liegt längs dem Umfang auf der Innenseite 8 der Schaftwand 7.

Das Material für das Band 18 ist derart gewählt, dass es anschmiegsam, elastisch dehnbar und flexibel ist.

Das Band 18 ist randseitig längs zweier streifenförmiger Bereich 19 und 21 flächig, mit der Innenseite 8 verklebt. Die streifenförmigen Bereiche 19 und 21 verlaufen im Abstand zueinander. Zwischen den Randstreifen 19 und 21 entsteht ein streifenförmiger Abschnitt 22, der sich aufgrund der Vorspannung des Bandes 18 nach innen vorwölbt.

Eine solche Konfiguration wird erhalten, wenn das Band 18 vor dem Einbau zu einer Manschette verklebt wird, deren Innendurchmesser im entspannten Zustand kleiner ist, als es dem Umfang des Querschnitts des Schaftes 2 auf der Höhe der Kammer 15 entspricht. Beim Ankleben der Randstreifen 19 und 21 bleibt der mittlere Bereich 22 nach innen zu vorgespannt.

Der Innenraum der Kammer 15 erstreckt sich auf diese Weise als geschlossener Ring, um den Innenraum 12 in Umfangsrichtung herum.

Die Schaftwand 7 enthält auf der Höhe der Kammer 15, zwischen den beiden Randstreifen 19 und 21 eine Bohrung 23, die als Druckausgleichskanal wirkt. Über die Bohrung 23 ist der Inneraum der Kammer 15 von der Außenatmosphäre her belüftet.

Der mittlere Bereich 22 hat gegenüber der Innenseite 8 eine Höhe b zwischen 0,5 und 3 cm, d.h. der Querschnitt des Innenraumes 12 verringert sich in diesem Bereich um 1 cm bis 6 cm.

Die Breite a des konvexen sich nach innen vorwölbenden Streifens 22 liegt zwischen 2 cm und 10 cm.

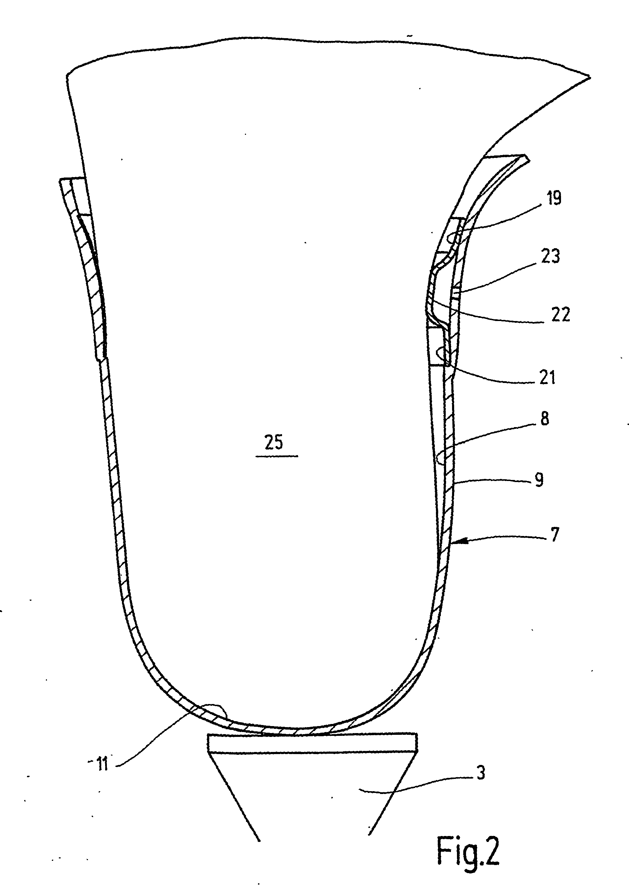

Die Wirkungsweise des neuen Prothesenschaftes 2 wird nunmehr in Verbindung mit Fig. 2 erläutert:

Beim Anziehen der Prothese steckt der Patient den amputierten Stumpf 25 von der Einstecköffnung 13 her in den Innenraum 12 des Prothesenschaftes 2. Dabei kann über das Ventil 14 die Luft entweichen. Das Einstecken ist beendet, sobald der Stumpf 25 nicht mehr tiefer in den Prothesenschaft 2 eintauchen kann.

Je nach Konizität des Prothesenstumpfes 25 wird früher oder später beim Einsteigen in den Prothesenschaft 2 die sich nach innen vorwölbende anschmiegsame Wand in Gestalt des sich vorwölbende Streifens 18 mit der Außenseite des Stumpfes 25 in Berührung kommen und den Innenraum der Kammer 15 verkleinert. Da das Material elastisch dehnbar ist, wird sich der streifenförmige Bereich 22 wie veranschaulicht, glatt und faltenfrei an der Außenseite des Stumpfes 25 anschmiegen.

Wenn oben stehend von Außenseite des Stumpfes 25 die Rede ist, kann es sich hierbei um die beispielsweise mit einem Hautpflegemittel versehen bloße Haut handeln, oder auch die Außenseite eines zuvor übergestreiften Liners.

Der Spaltraum der einerseits von dem Prothesenstumpf 25 und andererseits von der Innenseite 8 begrenzt wird, ist in Richtung auf die Einstecköffnung 13 durch den Prothesenstumpf 25 und den streifenförmigen Bereich 22, der an dem Prothesenstumpf 25 abdichtend anliegt, hermetisch luftdicht abgedichtet.

Wenn beim Tragen z.B. durch das Abheben der Beinprothese vom Boden und gegebenenfalls zusätzlich durch die Schwungbewegung beim Schreiten zwischen dem Stumpf 25 und dem Prothesenschaft 2 eine Kraft entsteht, die bestrebt ist, den Prothesenschaft 2 von dem Stumpf 25 abzuziehen, entsteht in dem vorerwähnten Spaltraum ein Unterdruck. Dieser Unterdruck ist auf der dem Stumpf benachbarten Seite des sich vorwölbenden und abdichtenden Bereichs 22 wirksam, während auf der gegenüberliegenden Seite im Bereich des Innenraums der Kammer 15 der Atmosphärendruck herrscht. Der streifenförmige Bereich 22 wird somit entsprechend dem Maß des Unterdrucks mit größerer Kraft gegen die Außenseite des Stumpfes 25 gepresst. Also bleibt die Abdichtwirkung zuverlässig erhalten.

Selbst wenn aufgrund der Gewebeelastizität ein unausgefüllter Spaltraum oder eine sehr hohe abziehende Kraft entsteht, bleibt die Abdichtwirkung erhalten. Die Anpresskraft erhöht sich mit der Erhöhung des Unterdrucks, unabhängig von der ursprünglichen Vorspannung mit der der streifenförmige elastische Bereich 22 zu Beginn an der Außenseite des Stumpfes 25 angelegen hat.

Die Anpresskraft, mit der der streifenförmige Bereich 22 im entlasteten Zustand anliegt, muss gerade eben ausreichen, um eine initiale Abdichtwirkung zustande zu bringen.

Wegen der geringen Vorspannung ist der Tragekomfort des erfindungsgemäßen Prothesenschaftes verbessert, weil im Bereich der die Abdichtung gewährleistenden Kammer 15 praktisch keine Radialkraft auf den Stumpf 25 ausgeübt wird. Die lichte Weite, die der vorspringende Streifen 22 frei lässt, muss gerade eben so groß sein, dass bei allen natürlich auftretenden Schwankungen des Volumens des Amputationsstumpfes im Bereich der Kammer 15 eine abdichtende Anlage aufrechterhalten bleibt.

Um aus dem Prothesenschaft 2 aussteigen zu können, muss der Benutzer das Ventil 14 öffnen, damit von unten her Luft in den Innenraum 12 nachströmen kann.

Die gezeigte Ausführungsform eignet sich sowohl für solche Amputationsstümpfe bei denen verhältnismäßig viel Gewebe vorhanden ist, beispielsweise Oberschenkelprothesen, als auch für Amputationsstümpfe, die relativ viel hartes Knochenmaterial unter der Haut aufweisen, wie beispielsweise Unterschenkelstümpfe.

Je nach der Art des spezifischen Stumpfes und den vorhanden Verhältnissen kann die Kammer, wie beschrieben, als geschlossener Ring ausgebildet sein, oder nur in jenen Bereichen an der Innenwand 8, in denen mit einer Undichtigkeit zu rechnen ist.

Es ist zweckmäßig, wenn die Kammer 15, d.h. die Abdichtung so dicht wie möglich an das proximale Ende herangerückt wird. Zwar ist an dieser Stelle die relative Volumenänderung und damit die relative Druckänderung kleiner verglichen mit einer distalen Lage der Kammer 15, aber die Redundanz in Falle von geringfügigen Leckagen ist günstiger, weil eine größere Luftmenge einströmen muss, ehe es zu einem merklichen Druckanstieg kommen kann, verglichen mit einer distalen Anordnung.

Bei dem Ausführungsbeispiel nach Fig. 1 wird die Kammer 15 zum Innenraum 12 quasi durch eine Manschette begrenzt, deren Ränder unter Vorspannung nach außen gezogen sind.

Es besteht auch die Möglichkeit die erforderliche Kammer dadurch zu erzeugen, dass Teile des Bandes 18 nach außen über den Rand der Einstecköffnung 13 umgestülpt sind.

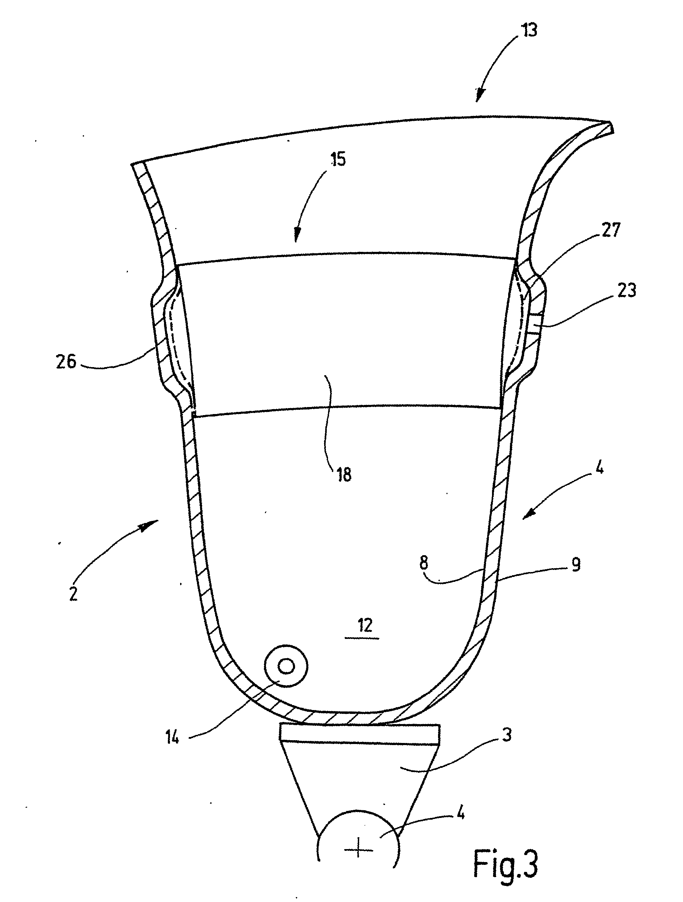

Fig. 3 zeigt eine alternative Ausführungsform die sich insbesondere für Prothesen eignet, die an Amputationsstümpfen sitzen, bei denen allseits sehr viel nachgiebiges Gewebe vorhanden ist.

Im Unterschied zu der Ausführungsform nach den Fig. 1 und 2 wird die Kammer 15 bei dem Ausführungsbeispiel nach Fig. 3 radial nach außen hin von einer ringförmigen Rille oder Nut begrenzt, die durch eine ringförmige oder rillenförmige radial nach außen gerichtete Ausstülpung 26 der schaftwand 7 gebildet ist. An der Innenseite entsteht eine längs dem Umfang umlaufende Ringnut 27, die von dem Band 18 aus elastischem Material überdeckt ist. Das Band 18 überspannt im Wesentlichen spannungsfrei die Ringnut 27 und ergänzt den Verlauf der Kontur der Innenseite 8 von distal nach proximal, so als wäre die Ringnut 27 nicht vorhanden.

Die so erhaltene Kammer 15 ist wiederum über die Bohrung 23 von der Außenatmosphäre her belüftet. Die Wirkungsweise der beschriebenen Oberschenkelprothese ist wie folgt:

Beim Einsteigen in den Prothesenschaft 2 wird das Gewebe des Amputationsstumpfes aufgrund der innenwohnenden Eigenelastizität das Band 18 ein Stück weit in den Ringraum radial nach außen drücken. Auf diese Weise wird sichergestellt, dass einerseits das Band 18 längs dem Umfang wie zuvor an dem Stumpf anliegt und andererseits jede Stelle des Kammerinnenraums das zwischen der Ringnut 27 und dem Streifen 18 begrenzt ist, über die Bohrung 23 belüftet ist.

Sollte eine Kraft bestrebt sein, den Prothesenschaft 2 von dem Stumpf abzuziehen, wird wie zuvor erläutert der Unterdruck wirksam, der in Verbindung mit dem Atmosphärendruck den hohlliegenden Bereich des Bandes 18 gegen den Amputationsstumpf andrückt und die Abdichtung bewerkstelligt.

Die Ausführungsform nach Fig. 3 hat den Vorteil der etwas einfachereren Herstellung, ist dafür aber auf solche Versorgungen beschränkt, bei denen der Amputationsstumpf allseitig viel nachgiebiges Gewebe aufweist. Sollte diese Bedingung nicht erfüllt sein, kann sie immer noch mit einem elastoplatischen Liner z.B. mit Gelfüllung erfüllt werden.

Der Grundgedanke der unterdruckabhängigen selbstverstärkenden Anpresskraft der Dichtung ist nicht nur auf Prothesenschäfte beschränkt, wie sie in den Fig. 1-3 gezeigt sind. Dieses Grundprinzip lässt sich auch bei Linern anwenden und ist somit zur Nachrüstung auch bestehender Prothesen geeignet.

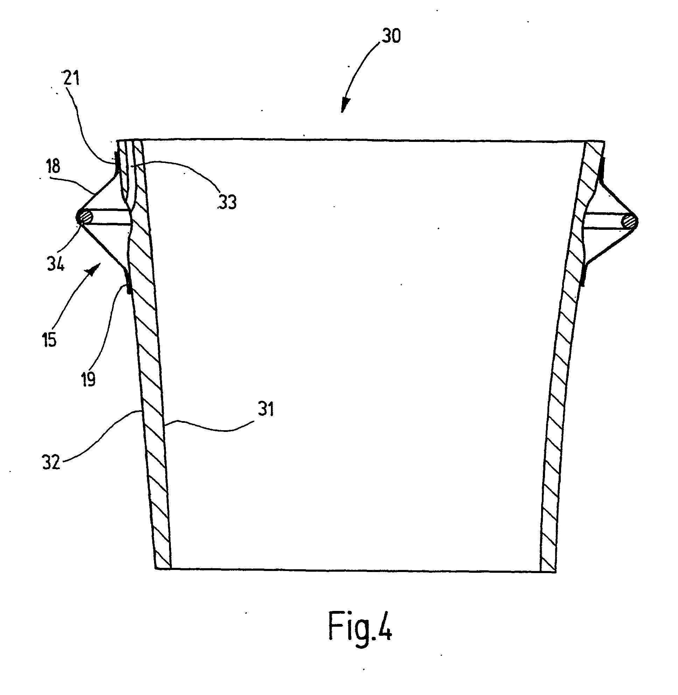

Fig. 4 zeigt einen erfindungsgemäßen Liner 30, der in Form einer nach unten offenen Manschette ausgeführt ist. Der Liner 30 besteht aus dem in der Prothetik üblichen Material, das einerseits hautverträglich ist und andererseits in radialer Richtung dehnbar ist.

Der Liner 30 bildet eine Manschette mit einer Innenseite 31 und einer Außenseite 32. An der Außenseite 32 ist in der Nähe des proximalen Endes die erwähnte Kammer 15 ausgebildet, die einerseits durch die Außenseite 32 des Liners 30 und andererseits durch das elastische dehnbare Band 18 begrenzt ist. Das Band 18 ist wiederum längs zweier Randstreifen 19 und 21 mit der Außenwand 32 im Wesentlichen luftdicht verklebt.

Der Druckausgleichskanal zum Belüften des Innenraums der Kammer 15 ist in Gestalt eines Kanals 33 in der Wand des Liners 30 ausgebildet und verbindet den Innenraum der Kammer 15 strömungsmäßig mit dem proximalen Rand des Liners 30. Der Druckausgleichskanal 33 kann durch zusätzliche Verstärkungselemente so gestaltet sein, dass er unabhängig von der Belastung des Liners 30 nicht verlegt werden kann. Um die initiale Abdichtkraft zu erzeugen, liegt in dem Innenraum der Kammer 15 ein elastischer Ring 34, der den mittleren Bereich des Bandes 18 radial nach außen vorspannt.

Die Darstellung in Fig. 4 ist nicht maßstäblich um das Wesentliche der Erfindung erkennen zu lassen.

Die Benutzung des erfindungsgemäßen Liners 30 sieht wie folgt aus: Der Patient zieht den manschettenartigen Liner 30 über den Amputationsstumpf bis auf die gewünschte Höhe. In diesem Zustand liegt die Innenseite 31 des Liners 30 satt und dichtend auf der Haut des Stumpfes auf. Sodann steigt der Patient mit dem Stumpf in den Prothesenschaft ein, beispielsweise einen Prothesenschaft, wie er in Fig. 1 gezeigt ist, dem jedoch die in Fig. 1 gezeigte Kammer 15 fehlt. Sobald der Stumpf tief genug in den Prothesenschaft eingedrungen ist, wird sich der nach außen vorwölbende Bereich des Bandes 18 an der Innenseite des Prothesenschaftes anlegen. Hierdurch kommt eine Abdichtung zwischen der Innenseite des Prothesenschaftes und dem elastisch nachgiebigen Band 18 zustande.

Die Abdichtwirkung aufgrund des entstehenden Unterdrucks und der Belüftung des Innenraums der Kammer 15 ist wie zuvor beschrieben.

Anstelle eines manschettenartigen Liners kann auch ein strumpfförmiger Liner mit geschlossenem distalem Ende verwendet werden.

Ein Prothesenschaft ist im Abstand zu dem distalen Ende mit einer ringförmig verlaufenden Kammer versehen. Die ringförmig verlaufende Kammer wird zur Innenseite des Prothesenschaftes von einer anschmiegsamen Wand gebildet, die dazu eingerichtete ist, sich abdichtend an der Außenseite eines Amputationsstumpfes anzulegen. Der Raum zwischen dieser anschmiegsamen Wand und dem harten Prothesenschaft ist von der Atmosphäre her belüftet, wodurch eine selbstverstärkende abdichtende Anpresskraft der anschmiegsamen Wand an den Stumpf zustande kommt, wenn im distalen Bereich des Prothesenschaftes zwischen der Wand und dem distalen Ende ein Unterdruck entsteht.

## Patentansprüche

1. Einen Stumpf aufnehmender becherförmiger Prothesenschaft (2),
mit einer im wesentlichen formstabilen Schaftwand (7), die eine nach außen weisende Außenseite (9) sowie eine nach innen weisende Innenseite (8) bildet,
mit einem im wesentlichen formstabilen Schaftboden (11), der mit der Schaftwand (7) verbunden ist und zusammen mit der Schaftwand (7) einen becherförmigen Innenraum (12) begrenzt, der zum Einführen des Stumpfes (25) über eine Einführöffnung (13) von außen zugänglich ist, die von einem Schaftrand begrenzt ist, wobei die Gestalt des Innenraums (13) an die Art des Stumpfes (25) und die Art der prothetischen Versorgung angepasst ist,
mit einer zum Innenraum (12) abgedichteten Kammer (15), die im Abstand von dem Schaftboden (11) an der Innenseite (8) der Schaftwand (7) angeordnet ist, sich zumindest ein Stück weit in Umfangsrichtung des Prothesenschaftes (2) erstreckt und eine zum Innenraum (12) des Prothesenschaftes (2) gelegene Wand (18) aus einem anschmiegsamen luftundurchlässigen Material aufweist, die als Dichtung zum Abdichten des Innenraums (12) gegen den Stumpf (25) dient,
**dadurch gekennzeichnet, dass** wenigstens ein Druckausgleichskanal (23) vorgesehen ist, der einends in die Kammer (15) einmündet und andernends ständig mit der Außenatmosphäre in Verbindung steht.

2. Prothesenschaft nach Anspruch 1, **dadurch gekennzeichnet, dass** die Kammer (15) sich als geschlossener Ring in Umfangsrichtung an der Innenseite (8) der Schaftwand (7) erstreckt.

3. Prothesenschaft nach Anspruch 1, **dadurch gekennzeichnet, dass** die zu der Außenseite (9) des Prothesenschaftes (2) hin gelegene Wand der Kammer (15) von der Schaftwand (7) gebildet ist.

4. Prothesenschaft nach Anspruch 1, **dadurch gekennzeichnet, dass** die Kammer (15) in Längsrichtung des Prothesenschaftes (2) eine Erstreckung zwischen 3 und 6 cm aufweist.

5. Prothesenschaft nach Anspruch 1, **dadurch gekennzeichnet, dass** das Material der anschmiegsamen Wand (18) elastisch dehnbar ist.

6. Prothesenschaft nach Anspruch 1, **dadurch gekennzeichnet, dass** der anschmiegsamen Wand (18) Mittel zugeordnet sind, die dazu eingerichtet sind, die anschmiegsame Wand (18) sich in den Innenraum (12) vorwölben zu lassen, und zwar über eine Strecke, die im wesentlichen der Länge der anschmiegsamen Wand (18) in Umfangrichtung des Innenraums (12) auf der Höhe der Kammer (15) entspricht.

7. Prothesenschaft nach Anspruch 1, **dadurch gekennzeichnet, dass** die Mittel von einer radialen Vorspannung der anschmiegsamen Wand (18) gebildet sind.

8. Prothesenschaft nach Anspruch 1, **dadurch gekennzeichnet, dass** die Kammer (15) derart gestaltet ist, dass die flexible Wand (18) eine Öffnung begrenzt, die in radialer Richtung 1 - 3 cm kleiner ist, als der Querschnitt des Innenraums (12) oberhalb oder unterhalb der Kammer (15).

9. Prothesenschaft nach Anspruch 1, **dadurch gekennzeichnet, dass** der Druckausgleichskanal von einer Bohrung (23) durch die Schaftwand (7) gebildet ist.

10. Prothesenschaft nach Anspruch 1, **dadurch gekennzeichnet, dass** der Prothesenschaft (2) auf der Höhe der Kammer (15) eine Vertiefung (26) enthält, die der Länge der Kammer (15) in Umfangsrichtung gesehen entspricht, und dass die Vertiefung (26) von der flexiblen Wand (18) verdeckt ist.

11. Prothesenschaft nach Anspruch 1, **dadurch gekennzeichnet, dass** die Vertiefung (26) von einer längs der Innenseite (8) sich erstreckenden Ringnut gebildet ist.

12. Prothesenschaft nach Anspruch 11, **dadurch gekennzeichnet, dass** er an allen Stellen einschließlich dem Bereich der Ringnut (26) im Wesentlichen gleiche Wandstärke aufweist.

13. Liner, der im Gebrauchszustand einen Stumpf zumindest manschettenartig umgibt, der eine nach außen weisende Außenseite (32) sowie eine nach innen weisende Innenseite bildet, die dem Stumpf zugekehrt ist, und der ein distales sowie ein proximales Ende aufweist,
mit einer auf der Außenseite (32) angeordneten Kammer (15), die im Abstand von dem distalen Ende angeordnet ist, sich zumindest ein Stück weit in Umfangsrichtung des Liners (30) erstreckt sowie eine nach außen gelegene und nach außen sich vorwölbende Wand (18) aus einem anschmiegsamen luftundurchlässigen Material aufweist, die als Dichtung zum Abdichten des Liners (30) gegen die Wand eines Prothesenschaftes dient, **dadurch gekennzeichnet, dass**
wenigstens ein Druckausgleichskanal (33) vorgesehen ist, der einends in die Kammer (15) einmündet, andernends ständig mit der Außenatmosphäre in Verbindung steht und der derart gelegen ist, dass er im Gebrauchszustand durchlässig bleibt.

14. Liner nach Anspruch 13, **dadurch gekennzeichnet, dass** die Kammer sich als geschlossener Ring in Umfangsrichtung längs der Außenseite (32) des Liners (30) erstreckt.

15. Liner nach Anspruch 13, **dadurch gekennzeichnet, dass** die zur Außenseite (32) des Liners (30) hin gelegene Wand der Kammer (15) von dem Liner (30) gebildet ist.

16. Liner nach Anspruch 13, **dadurch gekennzeichnet, dass** die Kammer (15) in Längsrichtung des Liners (30) eine Erstreckung zwischen 3 und 6 cm aufweist.

17. Liner nach Anspruch 13, **dadurch gekennzeichnet, dass** das Material der anschmiegsamen Wand (18) elastisch dehnbar ist.

18. Liner nach Anspruch 13, **dadurch gekennzeichnet, dass** der anschmiegsamen Wand (18) Mittel (34) zugeordnet sind, die dazu eingerichtet sind, die anschmiegsame Wand (18) sich radial nach außen vorwölben zu lassen, und zwar über eine Strecke, die im wesentlichen der Länge der anschmiegsamen Wand (18) in Umfangrichtung des Liners (30) auf der Höhe der Kammer (15) entspricht.

19. Liner nach Anspruch 13, **dadurch gekennzeichnet, dass** die Mittel von einem Spannring (34) gebildet sind, der in der Kammer (15) liegt.

20. Liner nach Anspruch 13, **dadurch gekennzeichnet, dass** die Kammer (15) derart gestaltet ist, dass die flexible Wand (18) im Gebrauchszustand einen Querschnitt definiert, der in radialer Richtung 1 - 3 cm größer ist, als der Querschnitt des Liners (30) oberhalb oder unterhalb der Kammer (15).

## Claims

1. Cup-shaped prosthesis shaft (2) for receiving a stump,
with a substantially dimensionally stable shaft wall (7), which forms an outwardly facing outer surface (9) as well as an inwardly facing inner surface (8),
with a substantially dimensionally stable shaft floor (11), which is joined to the shaft wall (7) and together with the shaft wall (7) delimits a cup-shaped interior (12), which is accessible from the outside for insertion of the stump (25) via an insertion opening (13), which is delimited by a shaft edge, wherein the shape of the interior (13) is matched to the type of stump (25) and the type of prosthetic care,
with a chamber (15), which is sealed towards the interior (12) and is spaced from the shaft floor (11) on the inner surface (8) of the shaft wall (7), extends at least a short distance in the peripheral direction of the prosthetic shaft (2) and has a wall (18) made of a supple airtight material, which is directed towards the interior (12) of the prosthetic shaft (2) and serves as a seal for sealing the interior (12) against the stump (25),
**characterised in that** at least one pressure equalising channel (23) is provided, which opens into the chamber (15) at one end and is in constant connection with the outside atmosphere at the other end.

2. Prosthetic shaft according to claim 1, **characterised in that** the chamber (15) extends in the peripheral direction as a closed ring on the inner surface (8) of the shaft wall (7).

3. Prosthetic shaft according to claim 1, **characterised in that** the wall of the chamber (15) directed towards the outer surface (9) of the prosthetic shaft (2) is formed by the shaft wall (7).

4. Prosthetic shaft according to claim 1, **characterised in that** the chamber (15) has an extent between 3 and 6 cm in the longitudinal direction of the prosthetic shaft (2).

5. Prosthetic shaft according to claim 1, **characterised in that** the material of the supple wall (18) is elastically extensible.

6. Prosthetic shaft according to claim 1, **characterised in that** the supple wall (18) has associated means arranged to enable the supple wall (18) to arch forwards into the interior (12), i.e. over a distance, which substantially corresponds to the length of the supple wall (18) in the peripheral direction of the interior (12) on the level of the chamber (15).

7. Prosthetic shaft according to claim 1, **characterised in that** the means are formed by a radial bias of the supple wall (18).

8. Prosthetic shaft according to claim 1, **characterised in that** the chamber (15) is configured in such a manner that the flexible wall (18) delimits an opening, which is 1-3 cm smaller in the radial direction than the cross-section of the interior (12) above or below the chamber (15).

9. Prosthetic shaft according to claim 1, **characterised in that** the pressure equalising channel is formed by a bore (23) through the shaft wall (7).

10. Prosthetic shaft according to claim 1, **characterised in that** at the level of the chamber (15) the prosthetic shaft (2) contains a depression (26), which corresponds to the length of the chamber (15) viewed in the peripheral direction, and that the depression (26) is covered by the flexible wall (18).

11. Prosthetic shaft according to claim 1, **characterised in that** the depression (26) is formed by an annular groove extending along the inner surface (8).

12. Prosthetic shaft according to claim 11, **characterised in that** it has a substantially equal wall thickness at all locations including the region of the annular groove (26).

13. Liner, which in the state of use surrounds a stump at least in a collar-like manner,
which forms an outwardly facing outer surface (32) as well as an inwardly facing inner surface, which is directed towards the stump, and which has a distal as well as a proximal end,
with a chamber (15), which is arranged on the outer surface (32) and is spaced from the distal end, extends at least a short distance in the peripheral direction of the liner (30) and also has an outwardly directed wall (18) made of a supple airtight material, which arches outwards and serves as a seal for sealing the liner (30) against the wall of a prosthetic shaft, **characterised in that**
at least one pressure equalising channel (33) is provided, which opens into the chamber (15) at one end and is in constant connection with the outside atmosphere at the other end and which is positioned in such a manner that it remains permeable in the state of use.

14. Liner according to claim 13, **characterised in that** the chamber extends in the peripheral direction as a closed ring along the outer surface (32) of the liner (30).

15. Liner according to claim 13, **characterised in that** the wall of the chamber (15) directed towards the outer surface (32) of the liner (30) is formed by the liner (30).

16. Liner according to claim 13, **characterised in that** the chamber (15) has an extent between 3 and 6 cm in the longitudinal direction of the liner (30).

17. Liner according to claim 13, **characterised in that** the material of the supple wall (18) is elastically extensible.

18. Liner according to claim 13, **characterised in that** the supple wall (18) has associated means (34) arranged to enable the supple wall (18) to arch radially outwards, i.e. over a distance, which substantially corresponds to the length of the supple wall (18) in the peripheral direction of the liner (30) on the level of the chamber (15).

19. Liner according to claim 13, **characterised in that** the means are formed by a tension ring (34), which lies in the chamber (15).

20. Liner according to claim 13, **characterised in that** the chamber (15) is configured in such a manner that in the state of use the flexible wall (18) defines a cross-section,
which is 1-3 cm larger in the radial direction than the cross-section of the liner (30) above or below the chamber (15).

## Revendications

1. Tige de prothèse (2) en forme de gobelet accueillant un moignon, et qui comprend :
- une paroi de tige (7) essentiellement indéformable, présentant une face externe (9) regardant vers l'extérieur et une face interne (8) regardant vers l'intérieur,
- un fond de tige (11) essentiellement indéformable qui est relié à la paroi de tige (7) en délimitant avec elle un volume interne (12) en forme de gobelet qui, pour l'introduction du moignon est accessible de l'extérieur par une ouverture d'introduction (13) délimitée par un bord de tige, la configuration du volume interne (13) étant adaptée au genre du moignon (25) et à celui de la prothèse fournie,
- fermée vers le volume interne (12), une chambre (15) qui est disposée à une certaine distance du fond de tige (11) sur la face interne (8) de la paroi de tige (7), présente au moins une certaine étendue selon la direction périphérique de la tige de prothèse (2) et possède, dirigée vers le volume interne (12) de la tige de prothèse (2), une paroi (18) qui est faite d'un matériau adaptable imperméable à l'air et qui sert à assurer l'étanchéité du volume interne (12) vis à vis du moignon (25),
cette tige étant **caractérisée en ce qu'**il est prévu au moins un canal d'équilibrage de pression (23) dont une extrémité débouche dans la chambre (15) tandis que l'autre extrémité est en liaison constante avec l'atmosphère, en étant placé de manière à être, en utilisation, ouvert.

2. Tige de prothèse selon la revendication 1, **caractérisée en ce que** la chambre (15) a la forme d'un anneau fermé s'étendant périphériquement le long de la face interne (8) de la paroi de tige (7).

3. Tige de prothèse selon la revendication 1, **caractérisée en ce que** la paroi de la chambre (15), du côté de la face externe (9) de la tige de prothèse, est constituée par la paroi de tige (7).

4. Tige de prothèse selon la revendication 1, **caractérisée en ce que** la chambre (15) présente, selon la direction longitudinale de la tige de prothèse (2) une longueur de 3 à 6 cm.

5. Tige de prothèse selon la revendication 1, **caractérisée en ce que** le matériau de la paroi adaptable (18) peut s'allonger élastiquement.

6. Tige de prothèse selon la revendication 1, **caractérisée en ce qu'**à la paroi adaptable (18) sont associés des moyens conçus pour l'amener à se bomber dans le volume interne (12) et cela sur une étendue qui correspond essentiellement à la longueur de cette paroi (18) en direction périphérique du volume interne (12) sur la hauteur de la chambre (15).

7. Tige de prothèse selon la revendication 1, **caractérisée en ce que** les moyens sont constitués par une précontrainte radiale appliquée à la paroi (18) adaptable.

8. Tige de prothèse selon la revendication 1, **caractérisée en ce que** la chambre (15) est configurée de manière que la paroi flexible (18) délimite une ouverture qui, en direction radiale est inférieure de 1 à 3 cm à la section du volume interne (12) au-dessus et en dessous de la chambre (15).

9. Tige de prothèse selon la revendication 1, **caractérisée en ce que** le canal d'équilibrage de pression est constitué par un perçage (23) traversant la paroi de tige (8).

10. Tige de prothèse selon la revendication 1, **caractérisée en ce que** la tige de prothèse (2) présente sur la hauteur de la chambre 15) une cavité (26) qui correspond en direction périphérique à la longueur de la chambre (15), cette cavité (26) étant recouverte par la paroi flexible (18).

11. Tige de prothèse selon la revendication 1, **caractérisée en ce que** la cavité (26) est constituée par une rainure annulaire qui s'étend le long de la face interne (8).

12. Tige de prothèse selon la revendication 11, **caractérisée en ce qu'**elle présente en tout point, y compris dans la zone de la rainure annulaire (8), essentiellement la même épaisseur.

13. Feuille qui en utilisation entoure un moignon au moins sous la forme d'une manchette, possède, regardant vers l'extérieur une face externe (32) et, regardant vers l'intérieur une face interne tournée vers le moignon, présente une extrémité distale et une extrémité proximale, avec, disposée sur la face externe (32), une chambre (15) éloignée de l'extrémité distale et qui présente au moins une certaine étendue selon la direction périphérique de la feuille (30) ainsi qu'une paroi (18) disposée vers l'extérieur et se bombant dans cette direction, qui est faite d'un matériau adaptable imperméable à l'air et qui sert à assurer l'étanchéité de la feuille (30) vis à vis de la paroi d'une tige de prothèse, cette feuille étant **caractérisée en ce qu'**il est prévu au moins un canal d'équilibrage de pression (33) dont une extrémité débouche dans la chambre (15) tandis que l'autre extrémité est en liaison constante
avec l'atmosphère, en étant placé de manière à être, en utilisation, ouvert.

14. Feuille selon la revendication 13, **caractérisée en ce que** la chambre a la forme d'un anneau fermé, s'étendant périphériquement le long de la face externe (32) de la feuille (30).

15. Feuille selon la revendication 13, **caractérisée en ce que** la paroi de la chambre (15) située du côté de la face externe (32) de la feuille est constituée par cette feuille.

16. Feuille selon la revendication 13, **caractérisée en ce que** la chambre (15) présente, selon la direction longitudinale de la feuille (30) une longueur de 3 à 6 cm.

17. Feuille selon la revendication 13, **caractérisée en ce que** le matériau de la paroi adaptable (18) peut s'allonger élastiquement.

18. Feuille selon la revendication 13, **caractérisée en ce qu'**à la paroi adaptable (18) sont associés des moyens conçus pour amener la paroi adaptable (18) à se bomber dans le volume interne (12) et cela sur une étendue qui correspond essentiellement à la longueur de cette paroi adaptable (18) en direction périphérique du volume interne (12) sur la hauteur de la chambre (15).

19. Feuille selon la revendication 13, **caractérisée en ce que** les moyens sont constitués par une bague de tension (34) disposée dans la chambre (15).

20. Feuille selon la revendication 13, **caractérisée en ce que** la chambre (15) est configurée de manière qu'en utilisation, la paroi flexible (18) définit une section qui en direction radiale est supérieure de 1 à 3 cm à celle de la feuille (30) au-dessus et en dessous de la chambre (15).
